# EUROPEAN PATENT APPLICATION

(11) **EP 3 235 563 A1**
(43) Date of publication of application: **25.10.2017**
(21) Application number: 16166226.7
(22) Date of filing: 20.04.2016
(51) Int. Cl.: B01J 19/08, C07C 2/80, C07C 9/06, C07C 11/04

(54) **A METHOD OF CONVERTING METHANE**

(71) Applicant: Shell Internationale Research Maatschappij B.V., 2596 HR Den Haag (NL)
(72) Inventor: SMITS, Jozef Jacobus Titus, 1031 HW Amsterdam (NL); VAN BAVEL, Alexander Petrus, 1031 HW Amsterdam (NL)
(74) Representative: Matthezing, Robert Maarten

(57) **Abstract**

The present invention provides a method of converting methane, the method at least comprising the steps of:
(a) providing a methane-containing stream;
(b) subjecting the methane-containing stream provided in step (a) in a first reactor to a cold plasma treatment, thereby obtaining a stream enriched in methyl radicals;
(c) transferring at least a part of the stream enriched in methyl radicals obtained in the first reactor in step (b) to a second reactor;
(d) subjecting the stream enriched in methyl radicals in the second reactor to a pressure increase, thereby at least obtaining a C2 hydrocarbon product.

## Description

The present invention relates to a method of converting methane.

Several methods for converting methane are known in the art.

As an example, WO9911572 discloses a process for the conversion of light hydrocarbons through the use of plasma and, in particular, through sliding electric discharges in the presence of oxygen and possibly water vapour.

As a further example, WO2010041113 discloses a process for the direct oxidation of methane to methanol in which the reaction is carried out in a gliding arc in tornado (GAT) reactor with cold plasma (also known as non-thermal plasma) technology.

Furthermore, WO2015025284 discloses a reactor comprising a plasma source and a catalyst comprising a mesoporous support material, and a process comprising feeding methane to said reactor in order to obtain one or more of ethene, hydrogen or carbon or downstream products derived from those products.

There has been a continuous desire in developing alternative processes that convert methane in more valuable products.

It is an object of the present invention to provide an alternative method for converting methane, in particular to obtain a C2 hydrocarbon product.

One or more of the above or other objects can be achieved by providing a method of converting methane, the method at least comprising the steps of:
(a) providing a methane-containing stream;
(b) subjecting the methane-containing stream provided in step (a) in a first reactor to a cold plasma treatment, thereby obtaining a stream enriched in methyl radicals;
(c) transferring at least a part of the stream enriched in methyl radicals obtained in the first reactor in step (b) to a second reactor;
(d) subjecting the stream enriched in methyl radicals in the second reactor to a pressure increase, thereby at least obtaining a C2 hydrocarbon product.

It has surprisingly been found according to the present invention that the conversion of methane into a C2 hydrocarbon product (such as ethane, ethene and ethyne), can be achieved using a cold plasma treatment, but in the absence of a catalyst, oxygen or both.

Also, it has surprisingly been found according to the present invention that the methyl radicals as obtained using the cold plasma treatment are stable enough to be transferred to a separate, second reactor.

Further, it has surprisingly been found that the method according to the present invention is also suited for methane-containing streams containing sulphur compounds, such as H₂S, mercaptans and COS.

In step (a), a methane-containing stream is provided. Although the methane-containing stream is not particularly limited, it is typically a methane-rich stream comprising at least 50 vol.% methane. Preferably, the methane-containing stream provided in step (a) comprises at least 90 vol.% methane, more preferably at least 95 vol.%, even more preferably at least 98 vol.%. Also, it is preferred that the methane-containing stream provided in step (a) comprises at most 2.0 vol.% O₂, preferably at most 1.0 vol.%, more preferably at most 0.5 vol.%, even more preferably at most 0.1 vol.%. As mentioned above, the method according to the present invention is also suited for methane-containing streams containing sulphur compounds. Hence, in an embodiment the methane-containing stream provided in step (a) comprises at least 0.1 wt.% S, and typically below 5.0 wt.% S.

In step (b), the methane-containing stream provided in step (a) is subjected in a first reactor to a cold plasma treatment, thereby obtaining a stream enriched in methyl radicals. As the person skilled in the art is familiar with cold plasma treatments (also called 'non-thermal plasma') this is not further discussed in detail. Cold plasma is distinguished from other plasma technologies, in that cold plasma is operated at or near room temperatures, whilst other plasmas operate at very high temperatures (such as above well above 2000°C). Examples of cold plasma treatments are plasma pencil, gliding arc, plasma needle, microwave-induced plasma and dielectric barrier discharge.

Typically, the methane-containing stream is subjected in step (b) to the cold plasma treatment at a temperature (defined here as the temperature of any solid element in the first reactor) of below 2000°C, preferably below 1000°C. Preferably, the methane-containing stream is subjected in step (b) to the cold plasma treatment at a temperature from 0°C to 500°C. Further it is preferred that the methane-containing stream is subjected in step (b) to the cold plasma treatment at a pressure below 5.0 bara, preferably below 2.0 bara, more preferably below 1.0 bara. Typically, the methane-containing stream is subjected to the cold plasma treatment at a pressure above 10 mbar.

In step (c), at least a part of the stream enriched in methyl radicals obtained in the first reactor in step (b) is transferred to a second reactor. Although the manner of transferring is not particularly limited, this is typically done by using a pressure difference (which can be achieved in various ways). It is of note that the first and second reactors are distinct reactors, and hence are e.g. not zones of one and the same reactor; in this respect it is noted that the pressure in the first reactor is preferably lower than the pressure in the second reactor (unless any intermediate step causes the pressure to drop between the first and the second reactor, before the pressure increase takes place in the second reactor). If desired, an intermediate step may take place between the first and the second reactor; as an example, separation of hydrogen radicals (if applicable) may take place between the first and second reactor, e.g. using absorption or a membrane.

In step (d), the stream enriched in methyl radicals is subjected in the second reactor to a pressure increase, thereby at least obtaining a C2 hydrocarbon product. Preferably, the pressure increase is at least 1.0 bar. A surprising advantage of the present invention is that the C2 hydrocarbon product may be obtained by pressure increase.

Preferably, in step (d) the pressure in the second reactor is increased to 0.5 bara to 10 bara, preferably to above 1.0 bara, more preferably to above 2.0 bara. Further it is preferred that in step (d) the temperature in the second reactor is from 0°C to 700°C, preferably above 10°C and preferably below 60°C. Although the person skilled in the art will readily understand that the pressure increase in the second reactor can be achieved in various ways, it is preferred according to the present invention that in step (d) the pressure is increased by feeding an inert gas to the second reactor. Preferably the inert gas is nitrogen or argon or a mixture thereof. Also, it is preferred that in step (d) the pressure is increased by reduction of the volume of the second reactor. If desired, the pressure may be increased using both feeding of an inert gas and reduction of volume.

According to an especially preferred embodiment according to the present invention, step (d) is performed in the absence of a catalyst. Also, it is particularly preferred that in step (d) no further reagents are added, in particular no O₂ and/or steam.

The person skilled in the art will readily understand that the C2 hydrocarbon product is not particularly limited, as long as it contains at least a compound having 2 carbon atoms. Preferably, the C2 hydrocarbon product obtained in step (d) comprises at least 1.0 vol.% of a compound having 2 carbon atoms. Preferably, the C2 hydrocarbon product comprises a compound that is selected from ethane, ethene and ethyne (acetylene) or a combination thereof.

According to an especially preferred embodiment, the C2 hydrocarbon product comprises:
- from 80 to 100 vol.% ethane; and
- from 0 to 20 vol.% ethene; and
- from 0 to 5 vol.% ethyne.

The person skilled in the art will understand that the C2 hydrocarbon product may be subjected to further reactions, if desired, in order to obtain other components.

Hereinafter the invention will be further illustrated by the following non-limiting example.

### Example

A methane-containing stream containing 99 vol.% methane and 1 vol.% H₂S is subjected in a first reactor to a cold plasma treatment using microwave discharge generation at a temperature of 1000°C and a pressure of 0.1 bara, thereby a stream enriched in methyl radicals.

The stream enriched in methyl radicals is then transferred from the first reactor to a second reactor using a pressure difference. The second reactor contains no catalyst and is free of oxygen (O₂). In the second reactor (temperature is 25°C), the stream enriched in methyl radicals is subjected to a pressure increase of 5 bar, by feeding nitrogen (N₂) gas to the second reactor. As a result of the pressure increase, a stream is obtained containing 1 vol.% ethane and ethene.

### Discussion

As can be seen from the Example, the present invention provides a method for converting methane into ethane and ethene, without using a catalyst and in the absence of oxygen (O₂) and steam.

The person skilled in the art will readily understand that many modifications may be made without departing from the scope of the invention.

## Claims

1. A method of converting methane, the method at least comprising the steps of:
(a) providing a methane-containing stream;
(b) subjecting the methane-containing stream provided in step (a) in a first reactor to a cold plasma treatment, thereby obtaining a stream enriched in methyl radicals;
(c) transferring at least a part of the stream enriched in methyl radicals obtained in the first reactor in step (b) to a second reactor;
(d) subjecting the stream enriched in methyl radicals in the second reactor to a pressure increase, thereby at least obtaining a C2 hydrocarbon product.

2. The method according to claim 1, wherein the methane-containing stream provided in step (a) comprises at least 90 vol.% methane, preferably at least 95 vol.%, more preferably at least 98 vol.%.

3. The method according to claim 1 or 2, wherein the methane-containing stream provided in step (a) comprises at most 2.0 vol.% O₂, preferably at most 1.0 vol.%, more preferably at most 0.5 vol.%, even more preferably at most 0.1 vol.%.

4. The method according to any of the preceding claims, wherein the methane-containing stream is subjected in step (b) to the cold plasma treatment at a temperature from 0°C to 500°C.

5. The method according to any of the preceding claims, wherein the methane-containing stream is subjected in step (b) to the cold plasma treatment at a pressure below 5.0 bara, preferably below 2.0 bara, more preferably below 1.0 bara.

6. The method according to any of the preceding claims, wherein in step (d) the pressure in the second reactor is increased to 0.5 bara to 10 bara, preferably to above 1.0 bara, more preferably to above 2.0 bara.

7. The method according to any of the preceding claims, wherein in step (d) the temperature in the second reactor is from 0°C to 700°C, preferably above 10°C and preferably below 60°C.

8. The method according to any of the preceding claims, wherein in step (d) the pressure is increased by feeding an inert gas to the second reactor.

9. The method according to any of the preceding claims, wherein in step (d) the pressure is increased by reduction of the volume of the second reactor.

10. The method according to any of the preceding claims, wherein step (d) is performed in the absence of a catalyst.

11. The method according to any of the preceding claims, wherein in step (d) no further reagents are added.

12. The method according to any of the preceding claims, wherein the C2 hydrocarbon product comprises:
- from 80 to 100 vol.% ethane; and
- from 0 to 20 vol.% ethene; and
- from 0 to 5 vol.% ethyne.
